# EUROPEAN PATENT SPECIFICATION

(11) **EP 2 166 874 B1**
(45) Date of publication and mention of the grant of the patent: **28.09.2016**
(21) Application number: 08771394.7
(22) Date of filing: 18.06.2008
(51) Int. Cl.: A23L 33/12, A23L 33/18, A61K 9/107, A61K 8/11, A61K 9/50

(54) **MICROENCAPSULATING COMPOSITIONS, METHODS OF MAKING, METHODS OF USING AND PRODUCTS THEREOF**
MIKROVERKAPSELUNGSZUSAMMENSETZUNGEN, VERFAHREN ZU IHRER HERSTELLUNG, VERFAHREN ZU IHRER VERWENDUNG UND IHRE PRODUKTE
COMPOSITIONS POUR MICROENCAPSULATION, LEURS PROCÉDÉS DE FABRICATION ET D'UTILISATION ET PRODUITS OBTENUS À PARTIR DE CELLES-CI

(30) Priority: 19.06.2007 US 945040 P
(43) Date of publication of application: 31.03.2010
(73) Proprietor: DSM IP Assets B.V., 6411 TE Heerlen (NL)
(72) Inventor: WILLS, Todd, Longmont Colorado, 80501 (US); CONNOLLY, Brian, J., Broomfield CO, 80020 (US); SUBRAMANIAN, Srinivasan, Broomfield, CO 80020 (US)
(74) Representative: Kurt, Manfred
(86) International application number: PCT/US2008/067383
(87) International publication number: WO 2008/157629

(56) References cited:
- WO-A1-01/74175
- US-A- 4 675 189
- US-A1- 2006 286 205
- US-B2- 6 974 592

## Description

### FIELD OF THE INVENTION

This invention relates to encapsulating compositions and methods of making them, as well as their use in making encapsulated compositions that contain core materials, including polyunsaturated fatty acids.

### BACKGROUND OF THE INVENTION

Encapsulation of compounds can protect them from undesirable chemical, physical, or biological change or breakdown while retaining their efficacy, such as biological or physiological efficacy. Encapsulation is also effective to improving the handling properties of a sticky material, provide for the controlled release of substances such as drugs or pesticides, masking the taste or odor of the compound, for instance. Microencapsulation of a liquid, such as an oil, allows the formation of a particle that presents a dry outer surface with an entrained oil. Often the particles are a free-flowing powder. Microencapsulation therefore effectively enables the conversion of liquids to powders. Microcapsules comprise roughly spherical particles that contain an encapsulated (entrapped) substance. The particle usually has some type of shell, often a polymeric shell, such as a polypeptide or polysaccharide shell, and the encapsulated active product is located within the shell. Certain compounds and compositions, such as polyunsaturated fatty acids (PUFAs), vitamins, minerals, antioxidants, hormones, amino acids, proteins, carbohydrates, coenzymes, and flavor agents, sensitive to any number of factors, can lose biological or other desired activity when unprotected. In addition, products (for example, decomposition products, degradation products, and oxidation products) that result from the chemical, physical, or biological change or breakdown of labile compounds and compositions, could lack the desired biological function and/or possess unwanted characteristics, such as having off-flavors, undesirable odors, irritation promoting activity and the like. There is often a need to introduce labile compounds and compositions, which are susceptible to chemical, physical, or biological change or breakdown, into pharmaceutical, nutritional, including nutraceutical, and industrial products. In such instances, protection of such compounds and compositions is desirable. With regard to PUFAs in particular, it is desirable to protect such lipids in food products from oxygen, trace metals and other substances which attack the double bonds of the PUFAs. Such protection reduces the likelihood of organoleptic problems, i.e., problems, relating to the senses (taste, color, odor, feel), such as off-flavors and undesirable odors, and other problems, such as loss of physiological activity, for instance. Such protection could potentially increase the shelf life of products containing them.

Numerous techniques for microencapsulation are known depending on the nature of the encapsulated substance and on the type of shell material used. Methods typically involve solidifying emulsified liquid droplets by changing temperature, evaporating solvent, or adding chemical cross-linking agents. Such methods include, for example, spray drying, interfacial polymerization, hot melt encapsulation, phase separation encapsulation (solvent removal and solvent evaporation), spontaneous emulsion, solvent evaporation microencapsulation, solvent removal microencapsulation, coacervation, and low temperature microsphere formation and phase inversion nanoencapsulation (PIN). Microencapsulation is suitable for drugs, vitamins and food supplements since this process is adaptable by varying the encapsulation ingredients and conditions.

There is a particular need to provide microencapsulated forms of fats or oils, such as vegetable and marine oils, which contain PUFAs. Such microencapsulated forms would benefit from the properties of digestibility, stability, resistance to chemical, physical, or biological change or breakdown. Microencapsulated oils could conveniently be provided as a free flowing powdered form. Such a powder can be readily mixed with other dry or liquid components to form a useful product.

The ability to microencapsulate, however, can be limited by factors due to the nature of the microencapsulation process or the compound or composition to be encapsulated. Such factors could include pH, temperature, uniformity, viscosity, hydrophobicity, molecular weight, and the like. Additionally, a given microencapsulation process may have inherent limitations. For example, in microencapsulation techniques in which heat is used for drying, low-boiling point aromatics can be lost during the drying process. Additionally, the core may adhere to the surface of the encapsulation material, presenting a potential for increased oxidation and changes in the flavor balance of the finished product. In some cases, storage conditions must be carefully controlled to avoid an increase in the water activity and therefore the stability of the capsule and retention of volatiles within the capsule. During spray drying microencapsulation, the feed inlet temperature may not be high enough and result in incomplete drying and sticking in the drying chamber or clump formation in storage. Particulate inconsistencies may also occur under some process conditions. At temperatures that are too low, the particles may balloon and cracks can form in the surface of the particles. This may cause loss of volatile compounds and compromise the quality of the final product. Yet another drawback is that the coatings produced are often water-soluble and temperature sensitive. The present inventors have recognized the foregoing problems and that there is a need, therefore, to provide additional materials and processes for encapsulation of compounds and compositions susceptible to chemical, physical, or biological change or breakdown.

US 2006/286205 discloses the preparation of a hydrolysate emulsion powder using enzymatic hydrolysis and browning reactions to modify the flavor of the resultant powder. Fermentation broth comprising Schizochytrium microorganisms was hydrolysed enzymatically with Alcalase, which results in partial hydrolysis. Thereafter, the mixture was heated to a temperature of 95°C and the pH brought to a value of 9.5. Once up to temperature, lactose, maltodextrin and high fructose corn syrup were added. This step inactivates the enzymes, promotes further protein hydrolysis, and the combination of protein hydrolysis products with the sugars results in browning reaction products (e.g. Maillard reaction products and caramelization products). This mixture was allowed to react for 2 hours then allowed to cool to 60°C. Upon cooling, Martek DHA oil was added, as well as casein. This mixture was homogenised at 750 bar in a Panda 2 k homogenizer (Niro-Soavi) maintaining a temperature of 60°C. The resultant broth was then spray-dried in a Buchi B-290 spray dryer with an inlet temperature of 170°C and an outlet temperature of 70°C.

WO 01/74175 A1 discloses a method of forming an emulsion of an oxygen sensitive oil by encapsulation thereof, which includes the steps of: a) preparing an aqueous mixture of a protein and a carbohydrate which contains a reducing sugar group; b) heating the mixture from 60°C to 160°C for a period to allow sufficient Maillard reaction products to form without coagulation; c) dispersing said oil in the aqueous phase; d) homogenising the mixture to obtain an emulsion. These Maillard reaction products formed with selected film forming proteinaceous materials produce superior encapsulants for oxygen sensitive oils or oil soluble ingredients. The protein is preferably soluble and needs to be stable in the heating range of the Maillard reaction and includes casein, soy and whey proteins, gelatin, egg albumin and hydrolyse proteins with increased free amino acid groups including soy protein hydrolysate. The preferred carbohydrate is a sugar with a reducing group preferably selected from the group consisting of monosaccharides (e.g. glucose, fructose), disaccharides (e.g. maltose, lactose), trisaccharides, oligosaccharides and glucose syrups. The pH of the aqueous phase is between 4 and 10. The oils include those that contain polyunsaturated fatty acids such as canola oil, borage oil, evening primrose oil, safflower oil, sunflower oil, flaxseed oil, wheat germ oil and grapeseed oil and marine oils obtained from fish such as tuna, herring, mackerel, sardine, cod liver, and shark. Dairy fats or other fats that are oxygen sensitive can also be encapsulated. Oil soluble ingredients that need protection from oxidation include vitamin A [retinol], vitamin D [calciferol], vitamin E, tocopherols, tocotrienols, vitamin K [quinone] and beta-carotene [pro-vitamin-A].

### SUMMARY OF THE INVENTION

The present invention provides a method for preparing an encapsulating composition, comprising reacting a solution comprising protein and reducing sugar at a starting pH of at least about 10 to achieve a degree of protein hydrolysis of between about 1% and about 15%, as claimed.

The invention further provides an encapsulated product, comprising a composition comprising a core material; and an encapsulant on the composition, wherein the encapsulant is formed from hydrolyzed protein having a degree of protein hydrolysis of between about 1% and about 15% and from caramelization products, as claimed.

The invention further provides a product selected from the group consisting of a food product, a cosmetic product, a pharmaceutical product, a nutraceutical product, and an industrial product, wherein the product comprises the encapsulated product described above, as claimed.

The invention also provides a method of preparing an encapsulated product, comprising reacting a solution comprising at least one protein and at least one reducing sugar at a starting pH of at least about 10 to achieve a degree of protein hydrolysis of between about 1% and about 15%; combining the reacted solution with a composition comprising a core material; wherein the reacted solution forms an encapsulant on the composition comprising the core material, as claimed.

The invention also provides encapsulated products prepared by this method, including a food product, a cosmetic product, a pharmaceutical product, a nutraceutical product, or an industrial product, as claimed.

In some embodiments, the hydrolyzed protein has an even distribution of hydrolysis products.

The protein is selected from the group consisting of casein, whey solids, whey protein isolate, soy protein. The reducing sugar is selected from the group consisting of glucose, and maltose.

In some embodiments, the degree of protein hydrolysis is between about 2% and about 10%, and in other embodiments, between about 3% and about 8%.

In some embodiments, caramelization products are produced during the step of reacting.

In some embodiments, Maillard reaction products are produced during the step of reacting.

In some embodiments, the reacting is at a temperature of at least about 90 °C for about one hour.

In some embodiments, the protein hydrolysis is non-enzymatic.

The encapsulant is formed by reacting a solution comprising protein and reducing sugar at a starting pH of at least about 10 to achieve a degree of protein hydrolysis of between about 1% and about 15%.

In some embodiments of the products, the encapsulant comprises caramelization products, and in other embodiments, the encapsulant comprises Maillard reaction products.

The core material can be a polyunsaturated fatty acid.

The plant can be an oilseed plant and/or crop plant. Where the plant is an oilseed plant, the source can be the oilseed of the oilseed plant.

The source of plant and/or oilseed can be soybean, corn, safflower, sunflower, canola, flax, peanut, mustard, rapeseed, chickpea, cotton, lentil, white clover, olive, palm, borage, evening primrose, linseed and tobacco or mixtures thereof.

The source can also be a genetically modified plant, a genetically modified oilseed, and a genetically modified microorganism, wherein the genetic modification preferably comprises the introduction of polyketide synthase genes.

The source can also be a microorganism selected from the group consisting of Thraustochytriales, dinoflagellates, and *Mortierella.* Thraustochytriales can include *Schizochytrium* and *Thraustochytrium.* The dinoflagellate can be of the genus *Crypthecodinium.*

The animal source can be an aquatic animal.

The core material is a polyunsaturated fatty acid selected from the group consisting of docosahexaenoic acid (DHA), docosapentaenoic acid (DPA), arachidonic acid (ARA), eicosapentaenoic acid (EPA), stearidonic acid (SDA), linolenic acid (LA), alpha linolenic acid (ALA), gamma linolenic acid (GLA), conjugated linolenic acid (CLA) and mixtures thereof.

In some embodiments of the products, the encapsulant is prepared by a method is selected from the group consisting of fluid bed drying, drum (film) drying, coacervation, interfacial polymerization, fluid bed processing, pan coating, spray gelation, ribbon blending, spinning disk, centrifugal coextrusion, inclusion complexation, emulsion stabilization, spray coating, extrusion, liposome nanoencapsulation, supercritical fluid microencapsulation, suspension polymerization, cold dehydration processes, and evaporative dispersion processes.

In some embodiments, a second and further encapsulants can be formed on the encapsulated product. In some embodiments, the second encapsulant is a prill coating and can be applied by prilling.

In some embodiments of the products, the product is insoluble in water, physically stable for at least about 30 days, and/or oxidatively stable for at least about 30 days.

In some embodiments of the products, the product has a particle size of between about 10 µm and about 3000 µm.

In some embodiments of the products, the product comprises core material in an amount between about 1 weight percent and about 50 weight percent.

In some embodiments of the products, the product is in a form selected from the group consisting of a free-flowing powder, a bead, a chip, and a flake.

The food product can be a liquid food product and/or a solid food product. Liquid food products include beverages, energy drinks, infant formula, liquid meals, fruit juices, liquid eggs, milk, milk products, and multivitamin syrups.

Food products include baby food, yogurt, cheese, cereal, powdered mixes, baked goods, food bars, and processed meats.

### BRIEF DESCRIPTION OF THE FIGURES

Figure 1 shows a schematic of the process described in Example 3.
Figure 2 shows the particle size distribution (volume vs. log particle size) after first and second pass homogenization for Emulsion #2 which is also representative for Emulsion 1 and 3. (◆ = emulsion #2, first pass; ▲= emulsion #2, second pass).
Figure 3 shows particle size distribution (volume vs. log particle size) of spray dried powders. Spray dried powder collected from cyclone for Powder 1 is also shown. (■ = Powder 1, collected from dryer main collection point; ▲ = Powder 1, collected from cyclone; ◆ = Powder 3, collected from dryer main collection point; O = Powder 3, collected from dryer main collection point).

### DETAILED DESCRIPTION OF THE INVENTION

The present invention provides encapsulating compositions and related methods for their preparation, as well as their use in making encapsulated compositions containing core materials. The encapsulating compositions of the present invention possess excellent film-forming properties for effective encapsulation. The resulting encapsulated products include highly stable powdered products with high loading capacities (i.e., ratio of product to the total composition of product plus encapsulant). As used herein, the term "a" or "an" refers to one or more of that entity; for example, a PUFA refers to one or more PUFAs or at least one PUFA. As such, the terms "a" (or "an"), "one or more" and "at least one" can be used interchangeably herein. It is also to be noted that the terms "comprising", "including", and "having" can be used interchangeably.

In a first embodiment, the invention provides a method for preparing an encapsulating composition by reacting a solution that contains protein and reducing sugar and that has a starting pH of at least about 10. As used herein, reacting refers to adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product. The solution is reacted to achieve a degree of protein hydrolysis of between about 1% and about 15%. The degree of hydrolysis may be determined in several ways, including analysis of the molecular weights of the hydrolyzed fragments or the distribution thereof, e.g. by size exclusion chromatography; determination of the viscosity of the preparation; determination of dispersibility of the preparation; determination of the amount of protein end-groups; or any combination of these and other suitable techniques. In general, it is not necessary to actually measure the degree of hydrolysis as the hydrolysis reaction is self-limiting. As explained in detail elsewhere herein, the hydrolysis reaction commences at a high starting pH and as the reaction proceeds, the pH drops and approaches neutral at which point the reaction stops.

Proteins that can be used to produce the encapsulating composition or encapsulated product include whey solids, whey protein isolate, soy protein, In some embodiments, the protein can be an enzyme-hydrolyzed protein. An enzyme-hydrolyzed protein can be prepared by methods known to those skilled in the art or can be obtained from a commercial source. It is worth pointing out that an enzyme-hydrolyzed protein is subjected to further hydrolysis as described herein (e.g., at high starting pH during a reacting step), but is generally not suitable as an encapsulant itself. Without being bound by theory, it is believed that a composition resulting from a protein hydrolysis as described herein contributes to the desirable features of the resulting encapsulated product. The hydrolysis of proteins at a high starting pH results in hydrolysis products having a uniform distribution of hydrolysis product lengths, since the sites of hydrolysis are more or less random. As used herein, protein hydrolysis products with a relatively uniform distribution of protein lengths refers to protein hydrolysis products that are produced by hydrolysis of a protein or peptide in a random manner. In contrast, enzyme-hydrolyzed proteins are not hydrolyzed randomly and do not result in a uniform distribution of hydrolysis product lengths. A composition having a uniform distribution of hydrolysis product lengths, when analyzed by SDS-PAGE, typically results in a smear of products from low to high molecular weights, with no particular fragment dominating. Enzyme-hydrolyzed proteins, on the other hand, typically show several dominant fragments on SDS-PAGE.

The uniform distribution of the molecular weights of the protein may be defined as follows: The hydrolyzed protein will have a molecular weight range. Over the range of molecular weights in a uniform distribution of hydrolysis products, if the range is divided into 10 equal parts, each part represents 5-15% of the sample or population.

In another embodiment, the hydrolyzed protein preferably has molecular weight distribution that is essentially equal to a distribution of molecular weights of hydrolyzed soy protein isolate obtained when soy protein isolate and carbohydrate are hydrated in water, the pH is adjusted to 10.5 -11.0 with NaOH and heated under reflux at 90-95°C for 60 minutes. In another embodiment, the molecular weight distribution is essentially equal to a distribution of molecular weights weights of hydrolyzed soy protein isolate obtained when protein is hydrated at a level of 5-15 % with carbohydrate, the pH is adjusted to 10.5 - 11.0 with NaOH and heated under reflux at 90-95°C for 60 minutes. In another embodiment, the molecular weight distribution is essentially equal to a distribution of molecular weights of hydrolyzed soy protein isolate obtained when protein is hydrated at a level of 8-12 % with carbohydrate, the pH is adjusted to 10.5 - 11.0 with NaOH and heated under reflux at 90-95°C for 60 minutes. In another embodiment, the molecular weight distribution is essentially equal to a distribution of molecular weights of hydrolyzed soy protein isolate obtained when protein is hydrated at a level of 10%. The uniform distribution of the molecular weights of the protein may be obtained my modifying the time and temperature of the reaction appropriately. As the temperature is reduced, the time of the reaction increases.

A reducing sugar is a sugar with a ketone or an aldehyde functional group, which allows the sugar to act as a reducing agent. In various embodiments, the reducing sugar can include glucose, and maltose. As used herein, the term reducing sugar also includes complex sources of reducing sugars. For example, suitable complex sources include corn syrup, corn syrup solids and modified starches such as chemically modified starches and hydrolyzed starches or dextrins, such as maltodextrin. Hydrolyzed starches (dextrins) are used in some embodiments. In some embodiments, the reducing sugar is formed *in situ* from, for example, a compound that is not itself a reducing sugar, but comprises reducing sugars. For example, starch is not a reducing sugar, but is a polymer of glucose, which is a reducing sugar. Hydrolysis of starch, by chemical or enzymatic means, yields glucose. This hydrolysis can take place *in situ,* to provide the reducing sugar glucose.

The relative amount of sugars and protein used to form the encapsulating material of the present invention can vary. In preferred embodiments, the ratio of sugar:protein can range from about 5:1 to about 1:5, from about 3:1 to about 1:3 and can be about 1:1. In addition, the total amount of sugar and protein in the solution can vary and can range up to the limits of solubility for a given set of conditions.

In some embodiments, the protein and reducing sugar are present in the same source material. For example, whey protein concentrate contains both protein and reducing sugar (in the form of lactose). Dried milk solids also contain protein and reducing sugar (in the form of lactose). A biomass hydrolysate or lysed cell mixture can also contain proteins and reducing sugars. In some cases, the reducing sugars are provided in the culturing media and are not fully metabolized by the microorganisms, and thus remain in the biomass hydrolysate or lysed cell mixture.

As used herein, a high starting pH is a pH of at least about 10, and can be any pH above about 10. For example, a starting pH of about 10.1, 10.2, 10.3, 10.4, 10.5, 10.6, 10.7, 10.8, 10.9, and so on can be used in the present invention. In addition, the starting pH typically can be below about 12, and in some embodiments, at or below about 11.

In various embodiments, the degree of protein hydrolysis achieved during the step of reacting can be between about 2% and about 10% or between about 3% and about 8%. Protein hydrolysis occurs in the process of the invention as a result of the pH and temperature conditions in processes of the present invention and therefore, is non-enzymatic hydrolysis.

In some embodiments, caramelization products are produced during the step of reacting. Caramelization refers to the thermal degradation of sugars leading to the formation of volatiles (generally resulting in a caramel aroma) and brown-colored products (which provide a caramel color). Like the Maillard reaction (see *infra*), caramelization is a type of non-enzymatic browning. The generation of flavors and colors in thermally induced caramelization requires that sugars, normally monosaccharide structures, first undergo intramolecular rearrangements. The reaction causes the release of H⁺ and the pH of the solution undergoing caramelization will therefore fall as the reaction proceeds. As noted above, the step of reacting in embodiments of the present invention take place at a starting pH of at least about 10. Over the course of the caramelization reaction, however, the pH will fall below about 10. In some embodiments, the caramelization reaction will proceed to completion. At completion, the pH of the reaction will approach neutral and will be in the range of about pH 6 to about pH 8.

In a further embodiment, the method for preparing an encapsulating composition or encapsulated product further comprises producing Maillard reaction products (MRPs) during the step of reacting. The Maillard reaction occurs when reducing sugars and amino acids react. The reducing sugar in the reaction can act as a reducing agent in the Maillard reaction. This reaction occurs in most foods on heating. Maillard reaction chemistry can affect desirable flavors and color of a wide range of foods and beverages. While not being bound by theory, it is believed that formation of MRPs in the products of the invention produces aromas and flavors that are desirable for inclusion in food products or other products that are consumed. MRPs can also possess antioxidant activity, and without being bound by theory, it is believed that this property of the MRPs imparts increased stability and shelf life to the products of the present invention. The Maillard reactions are well-known and from the detailed specification herein, temperature and time required to carry the reaction to the desired extent can be determined.

MRPs can be included in the methods and products of the present invention in a number of ways. In the process of reacting a solution that includes protein and reducing sugar, the temperature and time of reacting can be adjusted to promote the formation of MRPs. In general, the temperature of such a reaction can range from about 20°C to about 150 °C with from about 80 °C to about 110 °C being preferred. The time of the reaction can range from about 1 minute to about several hours, depending on the temperature. At the preferred higher temperature range, the time of reaction is preferably about 1 minute to about 20 minutes.

In a second embodiment, the invention provides a method for preparing an encapsulated product by preparing the encapsulating composition described above (by reacting a solution containing protein and reducing sugar and at a starting pH of at least about 10 to achieve a degree of protein hydrolysis of between about 1% and about 15%). The reacted solution is combined with a composition comprising a core material. This method further includes forming an encapsulant from the reacted solution on the composition comprising the core material.

The core material is a labile compound.

The labile compound is a PUFA. The PUFA can be docosahexaenoic acid C22:6(n-3) (DHA), omega-3 docosapentaenoic acid C22:5(n-3) (DPA), omega-6 docosapentaenoic acid C22:5(n-6) (DPA), arachidonic acid C20:4(n-6) (ARA), eicosapentaenoic acid C20:5(n-3) (EPA), stearidonic acid, linolenic acid, alpha linolenic acid (ALA), gamma linolenic acid (GLA), conjugated linolenic acid (CLA) or mixtures thereof. The PUFAs can be in any of the common forms found in natural lipids including but not limited to triacylglycerols, diacylglycerols, monoacylglycerols, phospholipids, free fatty acids, or in natural or synthetic derivative forms of these fatty acids (e.g. calcium salts of fatty acids, esters of fatty acids, including methyl esters, ethyl esters, and the like). Reference to an oil or other composition comprising an LC PUFA, as used in the present invention, can refer to either a composition comprising only a single LC PUFA such as DHA or a composition comprising a mixture of LC PUFAs such as DHA and EPA; or DHA and ARA; or DHA, EPA and ARA, etc.

PUFAs can be obtained from or derived from a plant (including oilseeds), a microorganism, an animal, or mixtures of the foregoing. The microorganisms can be algae, bacteria, fungi or protists. Microbial sources and methods for growing microorganisms comprising nutrients and/or PUFAs are known in the art (Industrial Microbiology and Biotechnology, 2nd edition, 1999, American Society for Microbiology). For example, the microorganisms can be cultured in a fermentation medium in a fermentor. PUFAs produced by microorganisms can be used in the methods and compositions of the present invention. In some embodiments, organisms include those selected from the group consisting of golden algae (such as microorganisms of the kingdom Stramenopiles), green algae, diatoms, dinoflagellates (such as microorganisms of the order Dinophyceae including members of the genus *Crypthecodinium* such as, for example, *Crypthecodinium cohnii*), yeast, and fungi of the genera *Mucor* and *Mortierella,* including but not limited to *Mortierella alpina* and *Mortierella* sect. *schmuckeri.* Members of the microbial group Stramenopiles include microalgae and algae-like microorganisms, including the following groups of microorganisms: Hamatores, Proteromonads, Opalines, Develpayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes (brown algae), Eustigmatophytes, Raphidophytes, Synurids, Axodines (including Rhizochromulinaales, Pedinellales, Dictyochales), Chrysomeridales, Sarcinochrysidales, Hydrurales, Hibberdiales, and Chromulinales. The Thraustochytrids include the genera *Schizochytrium* (species include *aggregatum, limnaceum, mangrovei, minutum, octosporum), Thraustochytrium* (species include *arudimentale, aureum, benthicola, globosum, kinnei, motivum, multirudimentale, pachydermum, proliferum, roseum, striatum*), *Ulkenia* (species include *amoeboidea, kerguelensis, minuta, profunda, radiate, sailens, sarkariana, schizochytrops, visurgensis, yorkensis*), *Aplanochytrium* (species include *haliotidis, kerguelensis, profunda, stocchinoi), Japonochytrium (species* include *marinum*)*, Althornia* (species include *crouchii*)*,* and *Elina* (species include *marisalba, sinorifica*)*.* The Labrinthulids include the genera *Labyrinthula* (species include *algeriensis, coenocystis, chattonii, macrocystis, macrocystis atlantica, macrocystis macrocystis, marina, minuta, roscoffensis, valkanovii, vitellina, vitellina pacifica, vitellina vitellina, zopfi), Labyrinthomyxa* (species include *marina*), *Labyrinthuloides* (species include *haliotidis, yorkensis*), *Diplophrys* (species include *archeri*)*, Pyrrhosorus** (species include *marinus*), *Sorodiplophrys** (species include *stercorea*), *Chlamydomyxa** (species include *labyrinthuloides, montana*)*.* (* = there is no current general consensus on the exact taxonomic placement of these genera).

Suitable microorganisms include those capable of producing lipids comprising the core materials omega-3 and/or omega-6 polyunsaturated fatty acids, and in particular microorganisms that are capable of producing DHA, DPA, EPA or ARA) will be described. More particularly, preferred microorganisms are algae, such as Thraustochytrids of the order Thraustochytriales, including *Thraustochytrium* (including *Ulkenia*) and *Schizochytrium* and including Thraustochytriales which are disclosed in commonly assigned U.S. Patent Nos. 5,340,594 and 5,340,742. More preferably, the microorganisms are selected from the group consisting of microorganisms having the identifying characteristics of ATCC number 20888, ATCC number 20889, ATCC number 20890, ATCC number 20891 and ATCC number 20892. Since there is some disagreement among experts as to whether *Ulkenia* is a separate genus from the genus *Thraustochytrium,* for the purposes of this application, the genus *Thraustochytrium* will include *Ulkenia.* Also preferred are strains of *Mortierella schmuckeri* (e.g., including ATCC 74371) and *Mortierella alpina.* Also preferred are strains of *Crypthecodinium cohnii,* including microorganisms having the identifying characteristics of ATCC Nos. 30021, 30334-30348, 30541-30543, 30555-30557, 30571, 30572, 30772-30775, 30812, 40750, 50050-50060, and 50297-50300. Oleaginous microorganisms are also preferred. As used herein, "oleaginous microorganisms" are defined as microorganisms capable of accumulating greater than 20% of the dry weight of their cells in the form of lipids. Genetically modified microorganisms that produce PUFAs are also suitable for the present invention. These can include naturally PUFA-producing microorganisms that have been genetically modified as well as microorganisms that do not naturally produce PUFAs but that have been genetically modified to do so.

Suitable organisms can be obtained from a number of available sources, including by collection from the natural environment. For example, the American Type Culture Collection currently lists many publicly available strains of microorganisms identified above. As used herein, any organism, or any specific type of organism, includes wild strains, mutants, or recombinant types. Growth conditions in which to culture or grow these organisms are known in the art, and appropriate growth conditions for at least some of these organisms are disclosed in, for example, U.S. Patent No. 5,130,242, U.S. Patent No. 5,407,957, U.S. Patent No. 5,397,591, U.S. Patent No. 5,492,938, and U.S. Patent No. 5,711,983.

Preferred microbial oils that are useful in the present invention include those that are disclosed in U.S. Patent Application Publication No. 2007-0003686 (entitled "Polyunsaturated Fatty Acid-Containing Oil Product and Uses and Production Thereof,"), Some of such oils are not subjected to winterization. A preferred microbial oil is known as Martek DHA™-HM and is produced by a process as disclosed in the foregoing patent applications, including a propanol and water extraction process that produces a product with a semi-solid characteristic.

Another source of PUFAs, in the compositions and methods of the present invention includes a plant source, such as oilseed plants. PUFA-producing plants, in alternate embodiments, can include those genetically engineered to express genes that produce PUFAs and those that produce PUFAs naturally. Such genes can include genes encoding proteins involved in the classical fatty acid synthase pathways, or genes encoding proteins involved in the PUFA polyketide synthase (PKS) pathway. The genes and proteins involved in the classical fatty acid synthase pathways, and genetically modified organisms, such as plants, transformed with such genes, are described, for example, in Napier and Sayanova, Proceedings of the Nutrition Society (2005), 64:387-393; Robert et al., Functional Plant Biology (2005) 32:473-479; or U.S. Patent Application Publication 2004/0172682. The PUFA PKS pathway, genes and proteins included in this pathway, and genetically modified microorganisms and plants transformed with such genes for the expression and production of PUFAs are described in detail in: U.S. Patent No. 6,140,486, U.S. Patent No. 6,566,583; U.S. Patent No. 7,247,461, U.S. Patent No. 7,211,418, U.S. Patent No. 7,217,856, U.S. Patent No. 7,271,315, PCT Publication No. WO 05/097982, and U.S. Patent No. 7,208,590.

Oilseed crops suitable for use in the present invention include soybeans, corn, safflower, sunflower, canola, flax, peanut, mustard, rapeseed, chickpea, cotton, lentil, white clover, olive, palm oil, borage, evening primrose, linseed, and tobacco that have been genetically modified to produce PUFA as described above.

Genetic transformation techniques for microorganisms and plants are well-known in the art. Transformation techniques for microorganisms are well known in the art and are discussed, for example, in Sambrook et al., 1989, Molecular Cloning: A Laboratory Manual, Cold Spring Harbor Labs Press. A general technique for transformation of dinoflagellates, which can be adapted for use with *Crypthecodinium cohnii,* is described in detail in Lohuis and Miller, The Plant Journal (1998) 13(3): 427-435. A general technique for genetic transformation of Thraustochytrids is described in detail in U.S. Patent Application Publication No. 20030166207, published September 4, 2003. Methods for the genetic engineering of plants are also well known in the art. For instance, numerous methods for plant transformation have been developed, including biological and physical transformation protocols. See, for example, Miki et al., "Procedures for Introducing Foreign DNA into Plants" in Methods in Plant Molecular Biology and Biotechnology, Glick, B.R. and Thompson, J.E. Eds. (CRC Press, Inc., Boca Raton, 1993) pp. 67-88. In addition, vectors and *in vitro* culture methods for plant cell or tissue transformation and regeneration of plants are available. See, for example, Gruber et al., "Vectors for Plant Transformation" in Methods in Plant Molecular Biology and Biotechnology, Glick, B.R. and Thompson, J.E. Eds. (CRC Press, Inc., Boca Raton, 1993) pp. 89-119. See also, Horsch et al., Science 227:1229 (1985); Kado, C.I., Crit. Rev. Plant. Sci. 10:1 (1991); Moloney et al., Plant Cell Reports 8:238 (1989); U.S. Patent No. 4,940,838; U.S. Patent No. 5,464,763; Sanford et al., Part. Sci. Technol. 5:27 (1987); Sanford, J.C., Trends Biotech. 6:299 (1988); Sanford, J.C., Physiol. Plant 79:206 (1990); Klein et al., Biotechnology 10:268 (1992); Zhang et al., Bio/Technology 9:996 (1991); Deshayes et al., EMBO J., 4:2731 (1985); Christou et al., Proc Natl. Acad. Sci. USA 84:3962 (1987); Hain et al., Mol. Gen. Genet. 199:161 (1985); Draper et al., Plant Cell Physiol. 23:451 (1982); Donn et al., In Abstracts of VIIth International Congress on Plant Cell and Tissue Culture IAPTC, A2-38, p. 53 (1990); D'Halluin et al., Plant Cell 4:1495-1505 (1992) and Spencer et al., Plant Mol. Biol. 24:51-61 (1994).

When oilseed plants are the source of PUFAs, the seeds can be harvested and processed to remove any impurities, debris or indigestible portions from the harvested seeds. Processing steps vary depending on the type of oilseed and are known in the art. Processing steps can include threshing (such as, for example, when soybean seeds are separated from the pods), dehulling (removing the dry outer covering, or husk, of a fruit, seed, or nut), drying, cleaning, grinding, milling and flaking. After the seeds have been processed to remove any impurities, debris or indigestible materials, they can be added to an aqueous solution and then mixed to produce a slurry. In some embodiments, milling, crushing or flaking is performed prior to mixing with water. A slurry produced in this manner can be treated and processed the same way as described for a microbial fermentation broth.

Another biomass source of nutrients, including PUFAs, in the compositions and methods of the present invention includes an animal source. Examples of animal sources include aquatic animals (e.g., fish, marine mammals, and crustaceans such as krill and other euphausids) and animal tissues (e.g., brain, liver, eyes, etc.) and animal products such as eggs or milk. Techniques for recovery of PUFA-containing oils from such sources are known in the art.

In the case of a whole cell, biomass, or oilseed, it will be recognized that these can include a PUFA, a vitamin or other beneficial compound. Whole cells and oilseeds include those described above as sources for PUFAs. As used herein, biomass can refer to multiple whole cells that, in the aggregate, constitute a biomass. A microbial biomass can refer to a biomass that has not been separated from the culture media in which the biomass organism was cultured. An example of a culture media is a fermentation broth. In a further embodiment, the biomass is separated from its culture = media by a solid/liquid separation prior to treatment by methods of the present invention. Typical solid/liquid separation techniques include centrifugation, filtration, and membrane filter pressing (plate and frame filter press with squeezing membranes). This (harvested) biomass usually has a dry matter content varying between 5% and 60%. If the water content is too high, the biomass can be dewatered by any method known in the art, such as, for example, spray drying, fluidized bed drying, lyophilization, freeze drying, tray drying, vacuum tray drying, drum drying, solvent drying, excipient drying, vacuum mixer/reactor drying, drying using spray bed drying, fluidized spray drying, conveyor drying, ultrafiltration, evaporation, osmotic dehydration, freezing, extrusion, absorbent addition or other similar methods, or combinations thereof. The drying techniques referenced herein are well known in the art. For example, excipient drying refers to the process of atomizing liquids onto a bed of material such as starch and solvent drying refers to a process where a solvent, miscible with water, is used in excess to replace the water. The biomass can optionally be washed in order to reduce extracellular components. The fermentation broth can be dried and then reconstituted to a moisture content of any desired level before treatment by any of the methods of the present invention.

The core materials of whole cells, biomass or oilseeds can be encapsulated using the methods of the present invention, as long as the whole cells, biomass or oilseeds are processed into a form that is physically suitable for encapsulation. Typically, this requires processing whole cells or biomass such that the average particle size is submicron, and preferably in the range of from about 0.1 µm to about 0.5 µm. Processing methods for oilseeds are described elsewhere herein. Additionally, hydrolyzing enzymes can be applied to dried biomass to form a biomass hydrolysate.

In a further embodiment, the encapsulated composition comprises an emulsified biomass hydrolysate. Such compositions and methods for making the same are described in detail in U.S. Provisional Patent Application Serial No. 60/680,740, filed on May 12, 2005; U.S. Provisional Patent Application Serial No. 60/781,430, filed on March 10, 2006; and U.S. Patent Application Serial No. 11/433,752, filed on May 12, 2006. Briefly, an emulsified biomass hydrolysate is obtained by hydrolyzing a nutrient-containing biomass to produce a hydrolyzed biomass, and emulsifying the hydrolyzed biomass to form a stable product. The stable product is typically an emulsion or a dry composition resulting from subsequent drying of the emulsion.

The step of forming an encapsulant from the reacted solution on the composition comprising the core material can be by any method known in the art. For example, the reacted solution and the composition comprising a core material can be spray-dried. Other methods for encapsulation are known, such as fluid bed drying, drum (film) drying, coacervation, interfacial polymerization, fluid bed processing, pan coating, spray gelation, ribbon blending, spinning disk, centrifugal coextrusion, inclusion complexation, emulsion stabilization, spray coating, extrusion, liposome nanoencapsulation, supercritical fluid microencapsulation, suspension polymerization, cold dehydration processes, evaporative dispersion processes, and methods that take advantage of differential solubility of coatings at varying temperatures.

Without intending to be bound by any theory, the encapsulant is believed to protect the composition comprising the core material to reduce the likelihood of or degree to which the core material undergoes a chemical, physical, or biological change or breakdown. The encapsulant can form a continuous coating on the composition comprising the core material (100% encapsulation) or alternatively, form a non-continuous coating (e.g., at a level that provides substantial coverage of the core material, for example, coverage at 80%, 90%, 95%, or 99% of the surface area). In other embodiments, the encapsulant can be a matrix in which the core material is entrapped.

Some exemplary encapsulation techniques are summarized below. It should be recognized that reference to the various techniques summarized below includes the description herein and variations of those descriptions known to those in the art. Other encapsulation techniques are also contemplated in the present invention.

In general, for microencapsulation of an oil product, such as a PUFA-containing oil described herein, it is important to form an emulsion of the oil by combining with the aqueous phase containing the coating materials. An emulsion containing oil droplets of about 200 nm in diameter, referred to as a fine emulsion, is generally desired for microencapsulation purposes. A coarse emulsion of oil in an aqueous phase, characterized by oil droplet sized of about 1000 nm in diameter can be prepared as an intermediate step to preparing a fine emulsion. A coarse emulsion can be prepared by known methods, including sonication and high shear mixing. Once a suitable emulsion is formed, one skilled in the art can determine appropriate microencapsulation methods based on emulsion characteristics such as solids content.

In spray drying, the core material to be encapsulated is dispersed or dissolved in a solution that includes a shell material. In food application, typically, the solution is aqueous and the solution includes a polymer or other shell material. Once a stable emulsion has formed, the solution or dispersion is pumped through a micronizing nozzle driven by a flow of compressed gas, and the resulting aerosol is suspended in a heated cyclone of air. The inlet temperature is often as high as possible without causing damage to the ingredients or other undesirable effects. Under these conditions, the atomized slurry forms micelles. The small size of the drops (averaging 100 micrometers in diameter) results in a relatively large surface area which dries quickly. As the water dries, the carrier forms a hardened shell around the core material. The dehydrated, solidified microparticles pass into a second chamber and are trapped in a collection flask.

Interfacial polycondensation is used to encapsulate a core material in the following manner. One monomer and the core material are dissolved in a solvent. A second monomer is dissolved in a second solvent (typically aqueous) which is immiscible with the first. An emulsion is formed by suspending the first solution in the second solution by stirring. Once the emulsion is stabilized, an initiator is added to the aqueous phase causing interfacial polymerization at the interface of each droplet of emulsion.

In hot melt encapsulation the core material is added to molten polymer. This mixture is suspended as molten droplets in a nonsolvent for the polymer (often oil-based) which has been heated to approximately 10°C above the melting point of the polymer. The emulsion is maintained through vigorous stirring while the nonsolvent bath is quickly cooled below the glass transition of the polymer, causing the molten droplets to solidify and entrap the core material.

In solvent evaporation encapsulation, a polymer is typically dissolved in a water immiscible organic solvent and the material to be encapsulated is added to the polymer solution as a suspension or solution in organic solvent. An emulsion is formed by adding this suspension or solution to a vessel of vigorously stirred water (often containing a surface active agent to stabilize the emulsion). The organic solvent is evaporated while continuing to stir. Evaporation results in precipitation of the polymer, forming solid microcapsules containing core material.

The solvent evaporation process is designed to entrap a liquid core material in a polymer, copolymer, or copolymer microcapsules. The polymer or copolymer is dissolved in a miscible mixture of solvent and nonsolvent, at a nonsolvent concentration which is immediately below the concentration which would produce phase separation (i.e., cloud point). The liquid core material is added to the solution while agitating to form an emulsion and disperse the material as droplets. Solvent and nonsolvent are vaporized, with the solvent being vaporized at a faster rate, causing the polymer or copolymer to phase separate and migrate towards the surface of the core material droplets. This phase separated solution is then transferred into an agitated volume of nonsolvent, causing any remaining dissolved polymer or copolymer to precipitate and extracting any residual solvent from the formed membrane. The result is a microcapsule composed of polymer or copolymer shell with a core of liquid material.

In solvent removal encapsulation, a polymer is typically dissolved in an oil miscible organic solvent and the material to be encapsulated is added to the polymer solution as a suspension or solution in organic solvent. An emulsion is formed by adding this suspension or solution to a vessel of vigorously stirring oil, in which the oil is a nonsolvent for the polymer and the polymer/solvent solution is immiscible in the oil. The organic solvent is removed by diffusion into the oil phase while continuing to stir. Solvent removal results in precipitation of the polymer, forming solid microcapsules containing core material.

In phase separation encapsulation, the material to be encapsulated is dispersed in a polymer solution by stirring. While continuing to uniformly suspend the material through stirring, a nonsolvent for the polymer is slowly added to the solution to decrease the polymer's solubility. Depending on the solubility of the polymer in the solvent and nonsolvent, the polymer either precipitates or phase separates into a polymer rich and a polymer poor phase. Under proper conditions, the polymer in the polymer rich phase will migrate to the interface with the continuous phase, encapsulating the core material in a droplet with an outer polymer shell.

Spontaneous emulsification involves solidifying emulsified liquid polymer droplets by changing temperature, evaporating solvent, or adding chemical cross-linking agents. Physical and chemical properties of the encapsulant and the material to be encapsulated dictates suitable methods of encapsulation. Factors such as hydrophobicity, molecular weight, chemical stability, and thermal stability affect encapsulation.

Coacervation is a process involving separation of colloidal solutions into two or more immiscible liquid layers (Dowben, R. General Physiology, Harper & Row, New York, 1969, pp. 142-143). Through the process of coacervation compositions comprised of two or more phases and known as coacervates may be produced. The ingredients that comprise the two phase coacervate system are present in both phases; however, the colloid rich phase has a greater concentration of the components than the colloid poor phase.

Low temperature microsphere formation has been described, see, e.g., U.S. Pat. No. 5,019,400. The method is a process for preparing microspheres which involves the use of very cold temperatures to freeze polymer-biologically active agent mixtures into polymeric microspheres. The polymer is generally dissolved in a solvent together with an active agent that can be either dissolved in the solvent or dispersed in the solvent in the form of microparticles. The polymer/active agent mixture is atomized into a vessel containing a liquid non-solvent, alone or frozen and overlayed with a liquefied gas, at a temperature below the freezing point of the polymer/active agent solution. The cold liquefied gas or liquid immediately freezes the polymer droplets. As the droplets and non-solvent for the polymer is warmed, the solvent in the droplets thaws and is extracted into the non-solvent, resulting in hardened microspheres.

Phase separation encapsulation generally proceeds more rapidly than the procedures described in the preceding paragraphs. A polymer is dissolved in the solvent. An agent to be encapsulated then is dissolved or dispersed in that solvent. The mixture then is combined with an excess of nonsolvent and is emulsified and stabilized, whereby the polymer solvent no longer is the continuous phase. Aggressive emulsification conditions are applied in order to produce microdroplets of the polymer solvent. After emulsification, the stable emulsion is introduced into a large volume of nonsolvent to extract the polymer solvent and form microparticles. The size of the microparticles is determined by the size of the microdroplets of polymer solvent.

Another method for encapsulating is by phase inversion nanoencapsulation (PIN). In PIN, a polymer is dissolved in an effective amount of a solvent. The agent to be encapsulated is also dissolved or dispersed in the effective amount of the solvent. The polymer, the agent and the solvent together form a mixture having a continuous phase, wherein the solvent is the continuous phase. The mixture is introduced into an effective amount of a nonsolvent to cause the spontaneous formation of the microencapsulated product, wherein the solvent and the nonsolvent are miscible.

In preparing an encapsulant of a composition comprising a core material, the conditions can be controlled by one skilled in the art to yield encapsulated material with the desired attributes. For example, the average particle size, hydrophobicity, biocompatibility, ratio of core material to encapsulant, thermal stability, and the like can be varied by one skilled in the art.

In some embodiments, this method further includes handling the core material under conditions that reduce oxidative degradation prior to encapsulation. Such handling can include, for example, maintaining the product in an inert atmosphere, the addition of antioxidants to the core material, and so forth.

Additional encapsulants, for example, a second encapsulant, a third encapsulant, a fourth encapsulant, a fifth encapsulant, and so on, are also contemplated in the present invention. Additional encapsulants can be applied by methods described herein, and can provide additional desirable properties to the products. For example, the additional encapsulants can further enhance the shelf life of the products, or modify the release properties of the product to provide for controlled release or delayed release of the core material. Without intending to be bound by theory, a second (or further) encapsulant is believed to further protect the composition comprising the core material to reduce the likelihood of or degree to which the core material undergoes a chemical, physical, or biological change or breakdown. The second encapsulant can form a continuous coating on the encapsulant (100% encapsulation) or alternatively, form a non-continuous coating (e.g., at a level that provides substantial coverage of the encapsulant, for example, coverage at 80%, 90%, 95%, or 99% of the encapsulant surface area). In other embodiments, the second encapsulant can be a matrix in which the encapsulant is entrapped.

The second encapsulant can be applied by any method known in the art, such as spray drying, fluid bed drying, drum (film) drying, coacervation, interfacial polymerization, fluid bed processing, pan coating, spray gelation, ribbon blending, spinning disk, centrifugal coextrusion, inclusion complexation, emulsion stabilization, spray coating, extrusion, liposome nanoencapsulation, supercritical fluid microencapsulation, suspension polymerization, cold dehydration processes, spray cooling/chilling (prilling), evaporative dispersion processes, and methods that take advantage of differential solubility of coatings at varying temperatures. While a second encapsulant can encapsulate a single discrete particle (i.e., a particle that is an encapsulant of a composition comprising a core material), a second encapsulant can alternatively encapsulate a plurality of discrete particles within a single second encapsulated particle. That is, the present invention contemplates a multi-core encapsulated product comprising a collection of primary encapsulated products, each primary encapsulated product comprising a core material and a first encapsulant on the core material; and a second encapsulant surrounding the collection.

In preferred embodiments, the second encapsulant of the encapsulant is a prill coating. Prilling is a process of encapsulating compounds in a high temperature melt matrix wherein the prilling material goes from solid to liquid above room temperature. Preferred materials and methods for prilling are disclosed in International Patent Application Publication No. WO 2007/150047, entitled "Encapsulated Labile Compound Compositions And Methods Of Making The Same".

In a third embodiment, the present invention also provides products that can be obtained by the methods of the invention. In one embodiment, the invention provides an encapsulated product that includes a composition comprising a core material; and an encapsulant on the composition. The encapsulant is formed from hydrolyzed protein having a degree of protein hydrolysis of between about 1% and about 15% and from caramelization products. Such a product can be prepared by reacting a solution comprising protein and reducing sugar at a starting pH of at least about 10 to achieve a degree of protein hydrolysis of between about 1% and about 15%, as claimed.

The products of the present invention can be is characterized in general by parameters such as particle size and distribution, particle geometry, active contents and distribution, release mechanism, and storage stability. In some embodiments in which the product is in a powder form, the product has a particle size of between about 10 µm and about 3000 µm, and in another embodiment between about 40 µm and 300 µm. Most particle sizing techniques assume that the material being measured is spherical. For irregularly shaped particles, the equivalent sphere approximation is useful in that it simplifies the way particle size distributions are represented, but different sizing techniques can produce different results. In the case of nonspherical particles the particle size can be reported as, for example, as the size of a sphere of the same maximum length, the size of a sphere of the same minimum length, the size of a sphere of the same weight, the size of a sphere of the same volume, the size of a sphere of the same surface area, the size of a sphere passing through the same sieve apparatus, and the size of a sphere with the same sedimentation rate. The products of the present invention are soluble in water; however, they can be made water insoluble by the addition of a second encapsulant such as a prill coating.

The products of the invention are generally physically stable. In some embodiments, the product is physically stable for at least about 30 days, at least about 60 days, at least about 90 days, at least about 120 days, at least about one year, at least about three years, or at least about five years. Physical stability refers to the ability of a product to maintain its physical appearance over time. For example, the structure of a product, with the encapsulant of the composition, is substantially maintained without, for example, the composition migrating through the encapsulant.

In various embodiments, the products of the invention are oxidatively stable. As used herein, oxidative stability refers to the lack of significant oxidation in the core material over a period of time. Oxidative stability of fats and oils can be determined by one skilled in the art. For example, peroxide values indicate the amount of peroxides present in the fat and are generally expressed in milli-equivalent oxygen per kg fat or oil. Additionally, anisidine values measure carbonyl (aldehydes and ketones) components which are formed during deterioration of oils. Anisidine values can be determined as described in IUPAC, Standard Methods for the Analysis of Oils, Fats and Derivatives, 6th Ed. (1979), Pergamon Press, Oxford, Method 2,504, page 143. The products of the invention, in some embodiments, have a peroxide value of less than about 2, or less than about 1. In other embodiments, products of the invention have an anisidine value of less than about 1. In some embodiments, the product is oxidatively stable for at least about 30 days, at least about 60 days, at least about 90 days, at least about 120 days, at least about one year, at least about three years, or at least about five years.

In other embodiments of the invention, the products have desirable aromas or flavors. In some embodiments, a desirable aroma or flavor is due to the presence of Maillard reaction products. In other embodiments, a desirable aroma or flavor, or lack of an undesirable aroma or flavor, is imparted to the product by the physical and oxidative stability of the product. The presence of desirable aromas and flavors can be evaluated by one skilled in the art. For example, the room-odor characteristics of cooking oils can be reproducibly characterized by trained test panels in room-odor tests (Mounts, J. Am. Oil Chem. Soc. 56:659-663, 1979). A standardized technique for the sensory evaluation of edible vegetable oils is presented in AOCS' Recommended Practice Cg 2-83 for the Flavor Evaluation of Vegetable Oils (Methods and Standard Practices of the AOCS, 4th Edition (1989)). The technique encompasses standard sample preparation and presentation, as well as reference standards and method for scoring oils. Panelists can be asked to rank the products on a Hedonic scale. Such a scale can be a scale of 1-10 used for the overall odor and flavor in which 10 is assigned to "complete blandness", and 1 to "strong obnoxiousness". The higher score will indicate better product in terms of aroma and flavor. In some embodiments, products of the present invention will have a score of at least about 5, at least about 6, at least about 7, at least about 8, at least about 9 or about 10 in such a test. Such evaluations can be conducted at various time frames, such as upon production of the product, at least about 60 days after production, at least about 90 days after production, at least about 120 days after production, at least about one year after production, at least about three years after production, or at least about five years after production.

Another scale for evalution of sensory properties is the Spectrum Scale for Intensity, a sensory scale for aromas and flavors that was developed by Sensory Spectra. Meilgaard, et al. (1999), SENSORY EVALUATION TECHNIQUES, 3rd ed., CRC Press, Florida; Appenidix 11.2., and further described in the Examples.

The amount of core material in the products of the invention will vary depending on the type of compound, the encapsulation materials used, and the methods used for forming the product. In some embodiments, the product comprises core material in an amount of at least about 1 to 20 weight percent, in 1% increments and up to about 40 to 80 weight percent, in 1% increments, for example, between about 1 weight percent and about 80 weight percent, between about 5 weight percent and about 70 weight percent, between about 10 weight percent and about 60 weight percent, between about 15 weight percent and about 50 weight percent, or between about 1 weight percent and about 50 weight percent.

The products of the present invention can be incorporated into nutritional products (including food products, food supplements, feed products, feed supplements, and nutraceutical products), cosmetic products, pharmaceutical products, and industrial products. Products can be in the form of chewable tablets, quick dissolve tablets, effervescent tablets, reconstitutable powders, elixirs, liquids, solutions, suspensions, emulsions, tablets, multi-layer tablets, bi-layer tablets, capsules, soft gelatin capsules, hard gelatin capsules, caplets, lozenges, chewable lozenges, beads, powders, granules, particles, dispersible granules, dietary supplements, genetically engineered designer foods, herbal products, and processed foods.

A nutritional product may be used directly as a food product, food supplement, feed product, feed supplement or as an ingredient in any of the foregoing. Food products can be liquid food products or solid food products. Liquid food products include, for example infant formula, liquid meals, liquid eggs, multivitamin syrups, meal replacers, medicinal foods, soups and syrups, and beverages. As used herein a beverage is any one of various liquids for drinking. Beverages include, for example, energy drinks, fruit juices, milk, and milk products. Solid food products include, for example, baby food, yogurt, cheese, cereal, powdered mixes, baked goods, including, for example, such items as cakes, cheesecakes, pies, cupcakes, cookies, bars, breads, rolls, biscuits, muffins, pastries, scones, and croutons, food bars including energy bars, and processed meats. Also included are doughs, batters, ice creams; frozen desserts; frozen yogurts; waffle mixes; salad dressings; and replacement egg mixes, baked goods such as cookies, crackers, sweet goods, snack cakes, pies, granola/snack bars, and toaster pastries; salted snacks such as potato chips, corn chips, tortilla chips, extruded snacks, popcorn, pretzels, potato crisps, and nuts; specialty snacks such as dips, dried fruit snacks, meat snacks, pork rinds, health food bars and rice/corn cakes; and confectionary snacks such as candy. In some embodiments, particularly including some solid food products, the product can be processed into a particulate form. For example, the particulate form can be selected from the group consisting of a bead, a chip, and a flake.

Feed or feed supplements can be prepared for any animal, including any companion animal or pet or for any animal whose products are consumed by humans. The term "animal" means any organism belonging to the kingdom Animalia and includes, without limitation, any animal from which poultry meat, seafood, beef, pork or lamb is derived. Seafood is derived from, without limitation, fish, shrimp and shellfish. Animal product includes any product derived from such animals, including, without limitation, meat, eggs, milk or other products. When fed to such animals, nutrients such as LC PUFAs can be incorporated into the flesh, milk, eggs or other products of such animals to increase their content of these nutrients.

A cosmetic product is a product that is applied to the skin and can function either to improve the appearance of the skin or to provide some dermatological benefit to the skin.

An industrial product is a product such as a raw material for manufacturing paints, wood products, textiles, adhesives, sealants, lubricants, leather, rope, paper pulp, plastics, fuels, oil, rubber working fluids, or metal working fluids.

Additional objects, advantages, and novel features of this invention will become apparent to those skilled in the art upon examination of the following examples thereof, which are not intended to be limiting.

### EXAMPLES

### Example 1

This example describes a general method for the preparation of the microencapsulated oils and Example 2 further describes preparation of specific microencapsulated oils.

Ingredient usage levels are outlined in Table 1. A protein source and a fraction of carbohydrate for caramelization are hydrated in water for 30 minutes at 55-60°C with constant agitation. Once ingredients are hydrated and fully dispersed, the pH is adjusted to 10.5-11.0 with NaOH. The adjusted solution is heated under reflux at 90-95°C for 60 minutes (time starts at 90°C). Following the hydrolysis / caramelization reaction, the solution is cooled and pH measured. If the pH is greater than 7, citric acid solution is added drop-wise until a final pH of approximately 7.0 is obtained. Finally, Martek DHA™-HM micro-algal oil derived from *Schizochytrium,* additional carbohydrate, and sodium ascorbate are added to the cooled solution and mixed for 3 minutes at 4,000 rpm to form coarse emulsion. Particle size of coarse emulsion is further reduced by homogenizing 1 pass at 500 bar. The emsulsion was spray dried at an inlet temperature of 180°C and an outlet temperature of 80°C by means of a Büchi 190 spray drying unit with two fluid nozzles.

**Table 1. Typical Ingredient List**

| **Ingredient** | **Usage Level (%)** |
|---|---|
| Water | 50 |
| Carbohydrate for caramelization (e.g., glucose) | 30 |
| Protein (e.g., soy protein isolate) | 10 |
| Martek DHA™-HM oil | 5 |
| Additional Carbohydrate (e.g., glucose) | 3.5 |
| Sodium Ascorbate | 1.5 |
| **Total** | **100%** |

### Example 2

This Example describes the preparation of microencapsulated oils by the method of Example 1 and variations of the method.
A. The method of Example 1 was performed with soy protein isolate (SPI) and glucose (a reducing sugar) to prepare microencapsulated Martek DHA™-HM oil.
B. The method of Example 1 to prepare microencapsulated Martek DHA™-HM oil was performed with SPI and glucose, except that the SPI was not hydrolyzed. That is, the caramelization reaction was performed in the absence of SPI. SPI, and additional carbohydrate were added after the caramelization step and cooling, but prior to the addition of the oil for emulsification.
C. The method of Example 1 to prepare microencapsulated Martek DHA™-HM oil was performed with SPI and sucrose (a non-reducing sugar).
D. The method of Example 1 to prepare microencapsulated Martek DHA™-HM oil was performed with a commercially available hydrolyzed soy protein isolate (HSPI) having 28% hydrolysis, and glucose.
E. Microencapsulated Martek DHA™-HM oil was prepared with SPI and glucose. This procedure was carried out similar to Example 1; except that hydrolysis protocol was accomplished with acid addition and sugar was removed from solution to determine if exogenous acid addition would catalyze protein hydrolysis. Soy protein isolate was hydrated, pH adjusted to 10.7 and heated (90-95°C) for 60 minutes. Throughout the heating step, a 10% citric acid solution was exogenously added. Care was taken to mimic the process of Example 1 in which it is believed acids are slowly formed during the hydrolysis/caramelization reaction. The pH was constantly monitored and at the end of the 60 minute heat cycle, the final pH was approximately 7.0. Following the modified procedure, the remaining ingredients were added.

### F. Results

Ultimate powder stability and characteristics of the method and alternatives are outlined below in Table 2.

**Table 2. Stability Characteristics of Select Prototypes.**

| **Example #** | **Sample** | **Free oil (%)** | **Induction Period (Weeks)** | |
|---|---|---|---|---|
| | | | **Head Space** | **Sensory** |
| 2A | SPI hydrolysis / Glucose | 0.71 | 11 | 9 |
| 2B | SPI no hydrolysis/ Glucose caramelization | 8.97 | 3 | 6 |
| 2C | SPI hydrolysis / Sucrose | 2.16 | 5 | 2 |
| 2D | Commercial HSPI (28% hydrolysis) / Glucose | N/A | N/A | N/A |
| 2E | SPI modified hydrolysis (acid addition) / Glucose | 1.10 | 2 | 7 |

| | | | | |
|---|---|---|---|---|
| Note: N/A: Unstable emulsion, no further processing | | | | |

Performance and stability of the final products were evaluated by measuring free oil, head space, and product (powder) sensory characteristics. Free oil is a quantitative measurement which demonstrates encapsulation efficiency. Efficiency is evaluated by determining the amount of easily extractable surface oil present in powder. Low free oil content indicates encapsulation ingredients and processes are sufficient in producing a highly uniform product; however, encapsulation efficiency alone cannot predict product stability. Since lipid oxidation is the largest contributing factor of a product's shelf stability, measurement of oxidation products provides a useful method of evaluating encapsulation matrices. Measurements of secondary oxidation products such as hexanal and propanal are widely used as markers to monitor oxidation of omega-3 fatty acids. Such measurements coupled with sensory analysis provide a reproducible method for accurately predicting shelf life. Head space results presented are derived from samples under accelerated storage conditions (40°C). Such conditions speed up reaction times, thus shortening amount of time required to evaluate shelf life. The head space induction period is defined by initial presence of oxidation products (propanal or hexanal), thus a higher induction period is indicative of a more oxidatively stable product.

Example 2A illustrates results typically obtained with the hydrolysis method of the present invention. The stability data in Table 2 indicates the functionality of the present invention. Results indicate powder performance is greatly improved when such method is employed. A direct comparison between Example 2A and Example 2B, which has the same ingredients as Example 2A but with no hydrolysis, demonstrates the effectiveness of hydrolysis procedure of the present invention. The non-hydrolyzed sample contains a much higher free oil content, as well as limited head space and sensory induction periods. Since samples were prepared with the same ingredients, differences in performance can be attributed to increased protein functionality imparted by the hydrolysis method.

Microencapsulated products prepared with the non-reducing sugar sucrose in Example 2C exhibit elevated free oil and limited stability. Loss of ingredient functionality in this example is directly related to lack of protein hydrolysis as indicated by lack of pH change. It is believed that due to the inability of sucrose to act as a reducing sugar, Maillard Browning and caramelization products acidic in nature are not formed. Lack of acidic product formation likely prevented the hydrolysis reaction from occurring.

When a commercially available hydrolyzed soy protein isolate characterized by a 28% degree of hydrolysis (DH) was substituted for soy protein in Example 2D, poor emulsion characteristics were observed. Upon shear mixing and subsequent homogenization, immediate separation occurred. Such emulsion instability prevents further processing as ingredients are not dispersed evenly. Lack of emulsion stability in this case is more than likely due to excessive protein hydrolysis. Without being bound by theory, the chemical hydrolysis method in Example 1 is believed to randomly cleave proteins resulting in uniform peptide size fragments. The limited nature and random cleavage of chemical hydrolysis results in a large peptide size fragment distribution, creating a complex interwoven network of different protein lengths when used as an emulsifier. Enzymatically hydrolyzed proteins are believed to contain much smaller peptides and an overall smaller peptide distribution than is found in the chemically hydrolyzed products. The functionality of such peptides would be greatly reduced as the ability of the smaller fragments to form a stable coating around oil droplets is affected.

A modified hydrolysis procedure as described in Example 2E was investigated to determine if exogenous acid addition would catalyze protein hydrolysis. Limited hydrolysis was achieved with the modified method; however, powder resulted in slightly higher free oil and lower induction periods. It is believed the complex reaction of protein hydrolysis, Maillard Browning, and caramelization as obtained using the method of Example 1 and 2A provides a unique coating system. Results indicate powders produced with the method of Example 2A display superior product characteristics when compared with the alternative methods investigated in Examples 2B-2E.

### Example 3

### A. Formulations

The formulations and calculated compositions of the emulsions for making spray dried powders are listed in Table 3.

**Table 3. Formulations and compositions of emulsions for making spray dried powders**

| **Formulation** | **#1 Emulsion (%)** | **#2 Emulsion (%)** | **#3 Emulsion (%)** | **Supplier** |
|---|---|---|---|---|
| DHA™-HM | 23.4 | 16.2 | 16.2 | Martek HM 75-4088 |
| Corn Oil | 0 | 7.2 | 0 | Mazola |
| Stable-Flake® | 0 | 0 | 7.2 | Cargill |
| Mono-glycerides | 0.234 | 0.234 | 0.234 | Danisco |
| Masking agent | 0.351 | 0.351 | 0.351 | Firmenich |
| Vitablend™ TAP1010 | 0 | 0.0288 | 0.0288 | Vitablend |
| Whey protein isolate BiPRO® | 4 | 4 | 4 | Davisco |
| | | | | |
| Maltose syrup 65% (caramelized) | 3.5 | 3.5 | 3.5 | Cargill |
| Maltose syrup 65% | 8 | 8 | 8 | Cargill |
| Glucose syrup 95% | 2.3 | 2.3 | 4.6 | Cargill |
| Sodium Ascorbate | 1.2 | 1.2 | 1.2 | Weisheng |
| Water | 57.015 | 56.986 | 54.686 | |
| **Total** | 100 | 100 | 100 | |

| **Calculated Emulsion Compositions** | **#1** | **#2** | **#3** | |
|---|---|---|---|---|
| Total solids (%) | 38.87 | 38.90 | 39.65 | |
| Total protein (%) | 3.60 | 3.60 | 3.60 | |
| Total fat (%) | 23.68 | 23.71 | 23.71 | |
| Total DHA (%) | 8.19 | 5.67 | 5.67 | |
| Total carbohydrate (%) | 11.45 | 11.45 | 12.20 | |
| Total ash (%) | 0.13 | 0.13 | 0.13 | |
| Total solids (%) - Measured | NA | 41.17 | 41.00 | |

As it is shown in Table 3, the overall oil load in the formulations is at 24%. The difference among the three formulations is in the amount of DHA™-HM oil. A small amount of TAP 1010 was added to #2 & 3 to compensate absence of the antioxidants in corn oil and Stable-flake S. The batch size of the emulsion was 230 kg. The total solids content of the emulsions was 40% before spray drying.

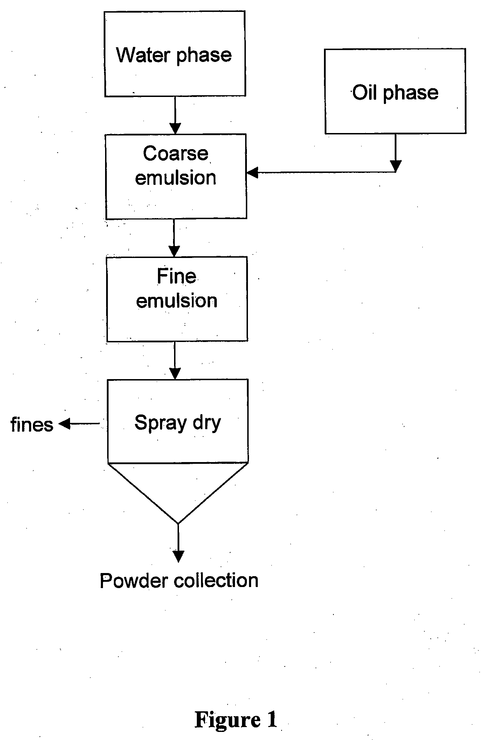

The basic steps performed on these formulations were 1) protein hydrolysis in a water phase containing water sugars, protein and sodium hydroxide, 2) mixing of oil phase containing DHA-containing microbial oil, monoglycerides, Vitablend™ TAP1010 (an antioxidant blend containing 10% mixed tocopherols, 10% ascorbyl palmitate, ,40% soy lecithin and 40% high oleic sunflower oil) and masking agent with the hydrolyzed water phase to form a coarse emulsion, 3) formation of a fine emulsion via homogenization, and 4) spray drying. The process diagram to illustrate the process is shown in Figure 1.

### B. Whey protein hydrolysis

Water, whey protein isolate and portions of maltose syrup were combined in the water phase tank. The protein was hydrated for 30 minutes at 55°C with slow mixing before the mixture pH was adjusted to 10.7 using 50% (w/w) sodium hydroxide. The mixture was then heated to 90-95°C and maintained at this temperature for 40-45 minutes before the heat was turned off. The process took approximately 1 hour to heat the mixture to temperature. After cooling, the mixture pH was approximately 7.2-7.5 at 60°C. Sodium ascorbate was added to further neutralize the pH. The remaining maltose and glucose syrup were added at this point. Citric acid was then added to lower the pH between 6.8 and 7.0 before combining with the oil phase. The actual amount of 50% NaOH and citric acid used can be found in Table 4.

**Table 4. Amount of sodium hydroxide and citric acid used for whey protein hydrolysis**

| **Emulsions** | **#1** | **#2** | **#3** |
|---|---|---|---|
| 50% (w/w) NaOH used (kg) | 1.18 | 1.22 | 1.12 |
| Citric Acid used (kg) | 0.436 | 0.410 | Not available |

### C. Formation of an emulsion.

Coarse emulsion. High shear mixing was performed on the mixture of the aqueous phase and oil phase for 15 minutes at 3550 rpm at about 65°C. Formulation #3 used Stable-flake® S in the formulation, which has a melting point of 70°C. During the 15 minutes high shear before homogenization, at the temperature of 60°C, the Stable-flake® S solidified and floated to the surface of the emulsion tank. This mixture was warmed to 71°C to re-melt the fat in the tank and to produce a homogeneous coarse emulsion before homogenization.

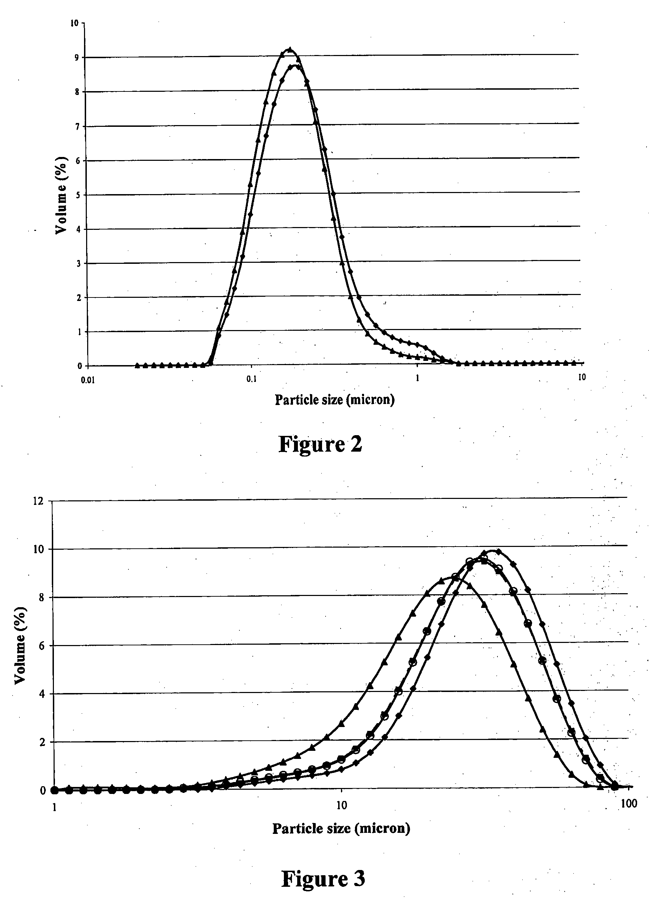

Fine emulsion. The coarse emulsion #1 was held in the tank at 60.5 °C for slightly longer than emulsion #2 or emulsion #3 before it was homogenized. A first pass homogenization was performed at approximately 65°C and 360 bar. A second pass homogenization was performed at approximately 45°C and 50 bar. The temperature can rise higher than these targets, thus, temperature is monitored carefully during this process. The particle size distributions of emulsion after first pass and second pass homogenization are shown in Table 5 and Figure 2. A second pass of emulsion through homogenization decreased the average particle size d(0.5) from 0.161 to 0.145 *µ*m and narrowed the size distribution compared to the particle size after the first pass. This should be beneficial to reduce the free oil content in spray dried powders and improve the powder stability.

**Table 5. Influence of number of homogenization passes on emulsion particle sizes**

| **Emulsions** | **#1** | **#2** | **#3** |
|---|---|---|---|
| ***Homogenization Pass*** | ***1^{st} Pass*** | ***1^{st} Pass*** | ***1^{st} Pass*** |
| Particle size d(0.5) | 0.163 | 0.161 | 0.157 |
| D[3,2] | 0.136 | 0.135 | 0.133 |
| D[4,3] | 0.223 | 0.221 | 0.219 |
| Uniformity | 0.719 | 0.719 | 0.734 |
| Temperature In (°C) | 72 | 66.1 | 76.7 |
| Temperature Out (°C) | 70.5 | 57.8 | 72.2 |
| ***Homogenization Pass*** | ***2^{nd} Pass*** | ***2^{nd} Pass*** | ***2^{nd} Pass*** |
| d(0.5) | Not available | 0.145 | 0.149 |
| D[3,2] | Not available | 0.122 | 0.129 |
| D[4,3] | Not available | 0.183 | 0.194 |
| Uniformity | Not available | 0.606 | 0.623 |
| Temperature In (°C) | 68.9 | 61.1 | 72.2 |
| Temperature Out (°C) | 59.4 | 55.6 | 63.9 |

| | | | |
|---|---|---|---|
| Note: 1. Instrument used for particle measurements was Malvern Mastersizer Hydro2000 2. d (0.5) - average mean diameter; D[3,2] - surface weighted mean diameter; D[4,3] - volume weighted mean diameter 3. Uniformity - a measure of the absolute deviations from the median 4. Temperature In/Out were actually temperature measured during homogenization | | | |

### D. Spray drying

After the emulsion was homogenized, it was put into a drum and transported to a spray-dryer. The emulsion was spray-dried at an inlet temperature of 150°C and an outlet temperature of 75°C by means of Niro Tall Form Spray Dryer™ capable of processing 500 kilograms of material per hour. The nozzle pressure was 138 bar with an SE60 nozzle and the flow rate was 7.5 kg/min.

For each run, approximately 72 kg of powder was collected from the dryer main collection point and from the cyclones. Powder collected from the main collection point was hot (65°C) when collected. Although the powders were put into a freezer as soon as possible after drying, this powder was subjected to more stress than powders that were cool when collected. The particle size distribution of powders can be found in Table 7 and Figure 3. As expected, the powder collected from cyclone is much finer than powder collected through the main collection point.

Upon drying, assuming the amount of Tricalcium Phosphate addition was 2% and the moisture content achieved was 3%.; the compositions of the 3 powders are shown in Table 6.

**Table 6. Calculated compositions of spray dried powder and yield**

| **Powder content - core (%)** | Powder #1 | Powder #2 | Powder #3 |
|---|---|---|---|
| Total moisture (%) | 3.00 | 3.00 | 3.00 |
| Total solids (%)-TCP | 95.00 | 95.00 | 95.00 |
| Total protein (%) | 8.81 | 8.80 | 8.63 |
| Total fat (%) | 57.88 | 57.90 | 56.81 |
| Total DHA (%) | 20.02 | 13.85 | 13.59 |
| Total carbohydrate (%) | 27.99 | 27.97 | 29.23 |
| TriCalcium Phosphate (TCP) | 2.00 | 2.00 | 2.00 |
| Total ash (%) - TCP | 0.32 | 0.32 | 0.32 |
| Total (%) | 100.00 | 100.00 | 100.00 |
| Theoretical yield of powder (kg) | 92.17 | 92.23 | 94.01 |
| Measured powder moisture content (%) | 3.04 | 2.89 | 2.68 |

**Table 7. Particle size distributions of spray dried powders**

| **Powders** | **Powder #1** | **Powder #1** | **Powder #2** | **Powder #3** |
|---|---|---|---|---|
| Code | 0514_1C | 0514_1C cyclone | 0514_2C | 0514_3C |
| Particle size d(0.1) | 27.062 | 17.33 | 32.865 | 27.038 |
| D(0.5) | 65.21 | 47.906 | 74.61 | 65.33 |
| D(3,2) | 47.86 | 30.823 | 56.37 | 47.23 |
| D(4,3) | 72.63 | 53.967 | 82.35 | 72.3 8 |
| Targeted d(0.5) | 60 | N/A | 60 | 60 |

| | | | | |
|---|---|---|---|---|
| Note: 1. d(0.1)-10% particle has particle size less than this diameter; d (0.5) - average mean diameter; D[3,2] - surface weighted mean diameter; D[4,3] - volume weighted mean diameter | | | | |

The analytical results of the spray-dried powders for DHA potency and free oil content are shown in Table 8. There are some discrepancies between the DHA potency content measured by different labs due to different extraction methods. All three powders had low free oil content (below 0.8%).

**Table 8. DHA potency and free oil content for spray dried powders**

| **Powder name** | **DHA content (mg/g)** | | | **Free oil** Measured (%) |
|---|---|---|---|---|
| | Expected | Measured (Lab 1) | Measured (Lab 2) | |
| **#1** | 200.2 | 201.70 | 205.2 | 0.72 |
| **#2** | 138.5 | 140.82 | 124.4 | 0.67 |
| **#3** | 135.9 | 138.23 | 162.0 | 0.57 |

Aromas were evaluated for three powders using the Spectrum Scale for Intensity, a sensory scale for aromas and flavors that was developed by Sensory Spectra. Meilgaard, et al. (1999), SENSORY EVALUATION TECHNIQUES, 3rd ed., CRC Press, Florida; Appenidix 11.2. Briefly, this is a scale from 0-15 for aromatics in which 0 represents none, 2 represents low, 5 represents low medium, 7.5 represents medium, 10 represents medium high, 12 represents high, and 15 represents very high. The results are shown in Table 9. No fishy notes were perceived; however, cardboard notes were noticed along with sweet caramel/brown sugar like smell. All three powders have similar aromas characteristics.

**Table 9. Sensory evaluation of spray dried powders (Aromas)**

| Attributes | Powder #1 | Powder #2 | Powder #3 |
|---|---|---|---|
| Total impact | 4.5 | 4.5 | 4.5 |
| Fishy | 0 | 0 | 0 |
| Cardboard// | 3 | 2 | 2 |
| Sweet (caramel/brown sugar like) | 1.5 | 2 | 2 |

A long-term sensory evaluation study was also performed on Powders 1-3. Panelists were asked to rate the odor on a scale from 1 to 5 with 3 being rancid and the lower score being better. A cutoff point of 3 was chosen as a reference for good sensory characteristic. Powders 1-3 had a sensory score of less than 3 for 6 weeks, indicating the ability of the powder to protect the oil components from oxidation and other reactions that cause off flavors.

### E. Stability study at 40°C

Each powder was placed in a vial for GC headspace analysis and stored at 40°C for several weeks. The headspace of each sample then was analyzed by GC to determine the amount of test compounds indicative of oxidation of PUFAs in the DHA-containing oil. The five test compounds chosen were: propanal, 2-pentenal, hexanal, 1-octa-3-one, and 1-octen-3-ol. The headspace test compounds were analyzed by headspace gas chromatography on a weekly basis. A cutoff point of 1 ppm for total volatiles (test compounds) was chosen as a reference. Powders 1-3 had less than 1 ppm total volatiles for 16-17 weeks, indicating the ability of the powder to protect the oil components from oxidation.

In summary, the powders made had and average size d(0.5) 60 microns, free oil content below 0.8%. The three powders had similar sensory properties, including cardboard along with sweet/brown sugar-like smells with the overall impact score of 4.5. Furthermore, the powders are stable to oxidation. This process is robust and can be scaled up.

## Claims

1. A method of preparing an encapsulated product, comprising
reacting a solution comprising at least one protein selected from the group consisting of whey solids, whey protein isolate and soy protein and at least one reducing sugar selected from the group consisting of glucose and maltose at a starting pH of at least about 10 to achieve a degree of protein hydrolysis of between about 1 % and about 15%, wherein the reacting is at a temperature of at least 90°C;
combining the reacted solution with a composition comprising a core material;
wherein the reacted solution forms an encapsulant on the composition comprising the core material, and wherein
the core material comprises a polyunsaturated fatty acid selected from the group consisting of docosahexaenoic acid (DHA), docosapentaenoic acid (DPA), arachidonic acid (ARA), eicosapentaenoic acid (EPA), stearidonic acid (SDA), linolenic acid (LA), alpha linolenic acid (ALA), gamma linolenic acid (GLA), conjugated linolenic acid (CLA) and mixtures thereof.

2. The method of claim 1, wherein the degree of protein hydrolysis is between about 2% and about 10%.

3. The method of any of claims 1-2, wherein the reacting is for about one hour.

4. The method of any of claims 1-3, wherein the protein hydrolysis is non-enzymatic.

5. The method of any of claims 1-4, wherein the starting pH is selected from the group consisting of a pH between 10 and 14, a pH between 11 and 12, and a pH between 10 and 11.

6. The method of any of claims 1-5, further comprising applying a second encapsulant to the encapsulated product.

7. The method of claim 6, wherein the applying a second encapsulant is prilling.

8. An encapsulated product, comprising:
a composition comprising a labile compound core material selected from the group consisting of a polyunsaturated fatty acid selected from the group consisting of docosahexaenoic acid (DHA), docosapentaenoic acid (DPA), arachidonic acid (ARA), eicosapentaenoic acid (EPA), stearidonic acid (SDA), linolenic acid (LA), alpha linolenic acid (ALA), gamma linolenic acid (GLA), conjugated linolenic acid (CLA) and mixtures thereof; and
an encapsulant on the composition, wherein the encapsulant is formed from hydrolyzed protein having a degree of protein hydrolysis of between about 1% and about 15% and from caramelization products,
wherein the encapsulant is obtainable by reacting a solution comprising protein selected from the group consisting of whey solids, whey protein isolate **and** soy protein, and reducing sugar selected from the group consisting of glucose and maltose at a starting pH of at least about 10 to achieve a degree of protein hydrolysis of between about 1 % and about 15%, and the reacting is at a temperature of at least about 90°C.

9. The encapsulated product of claim 8, wherein the reacting is for at least about one hour.

10. The encapsulated product of any of claims 8-9, wherein the starting pH is selected from the group consisting of a pH between 10 and 14, a pH between 11 and 12, and a pH between 10 and 11.

11. The encapsulated product of any of claims 8-10, wherein the degree of protein hydrolysis is between about 2% and about 10%.

12. The encapsulated product of any of claims 8-11, wherein the encapsulant comprises Maillard reaction products.

13. A product selected from the group consisting of a food product, a cosmetic product, a pharmaceutical product, a nutraceutical product, and an industrial product, wherein the product comprises the product of any of claims 8-12.

## Patentansprüche

1. Verfahren zur Herstellung eines verkapselten Produkts, umfassend
Umsetzen einer Lösung, die wenigstens ein aus der aus Molkefeststoffen, Molkeproteinisolat und Sojaprotein bestehenden Gruppe ausgewähltes Protein und wenigstens einen aus der aus Glucose und Maltose bestehenden Gruppe ausgewählten reduzierenden Zucker umfasst, bei einem Ausgangs-pH-Wert von wenigstens etwa 10, so dass ein Proteinhydrolysegrad zwischen etwa 1% und etwa 15% erzielt wird, wobei das Umsetzen bei einer Temperatur von wenigstens 90°C erfolgt;
Kombinieren der umgesetzten Lösung mit einer ein Kernmaterial umfassenden Zusammensetzung;
wobei die umgesetzte Lösung eine Verkapselung auf der das Kernmaterial umfassenden Zusammensetzung bildet und wobei
das Kernmaterial eine mehrfach ungesättigte Fettsäure umfasst, die aus der aus Docosahexaensäure (DHA), Docosapentaensäure (DPA), Arachidonsäure (ARA), Eicosapentaensäure (EPA), Stearidonsäure (SDA), Linolensäure (LA), Alpha-Linolensäure (ALA), Gamma-Linolensäure (GLA), konjugierter Linolensäure (CLA) und Gemischen davon bestehenden Gruppe ausgewählt ist.

2. Verfahren nach Anspruch 1, wobei der Proteinhydrolysegrad zwischen etwa 2% und etwa 10% liegt.

3. Verfahren nach einem der Ansprüche 1-2, wobei das Umsetzen etwa eine Stunde lang erfolgt.

4. Verfahren nach einem der Ansprüche 1-3, wobei die Proteinhydrolyse nichtenzymatisch ist.

5. Verfahren nach einem der Ansprüche 1-4, wobei der Ausgangs-pH-Wert aus der aus einem pH-Wert zwischen 10 und 14, einem pH-Wert zwischen 11 und 12 und einem pH-Wert zwischen 10 und 11 bestehenden Gruppe ausgewählt ist.

6. Verfahren nach einem der Ansprüche 1-5, ferner umfassend Auftragen einer zweiten Verkapselung auf das verkapselte Produkt.

7. Verfahren nach Anspruch 6, wobei es sich bei dem Auftragen einer zweiten Verkapselung um Sprühkondensation handelt.

8. Verkapseltes Produkt, umfassend:
eine Zusammensetzung, die ein Labile-Verbindung-Kernmaterial umfasst, das aus der aus einer mehrfach ungesättigten Fettsäure, die aus der aus Docosahexaensäure (DHA), Docosapentaensäure (DPA), Arachidonsäure (ARA), Eicosapentaensäure (EPA), Stearidonsäure (SDA), Linolensäure (LA), Alpha-Linolensäure (ALA), Gamma-Linolensäure (GLA), konjugierter Linolensäure (CLA) und Gemischen davon bestehenden Gruppe ausgewählt ist, bestehenden Gruppe ausgewählt ist; und
eine Verkapselung auf der Zusammensetzung, wobei die Verkapselung aus hydrolysiertem Protein mit einem Proteinhydrolysegrad zwischen etwa 1% und etwa 15% und aus Karamelisierungsprodukten gebildet wird,
wobei die Verkapselung durch Umsetzen einer Lösung, die aus der aus Molkefeststoffen, Molkeproteinisolat und Sojaprotein bestehenden Gruppe ausgewähltes Protein und aus der aus Glucose und Maltose bestehenden Gruppe ausgewählten reduzierenden Zucker umfasst, bei einem Ausgangs-pH-Wert von wenigstens etwa 10, so dass ein Proteinhydrolysegrad zwischen etwa 1% und etwa 15% erzielt wird, erhältlich ist und das Umsetzen bei einer Temperatur von wenigstens 90°C erfolgt.

9. Verkapseltes Produkt nach Anspruch 8, wobei das Umsetzen wenigstens etwa eine Stunde lang erfolgt.

10. Verkapseltes Produkt nach einem der Ansprüche 8-9, wobei der Ausgangs-pH-Wert aus der aus einem pH-Wert zwischen 10 und 14, einem pH-Wert zwischen 11 und 12 und einem pH-Wert zwischen 10 und 11 bestehenden Gruppe ausgewählt ist.

11. Verkapseltes Produkt nach einem der Ansprüche 8-10, wobei der Proteinhydrolysegrad zwischen etwa 2% und etwa 10% liegt.

12. Verkapseltes Produkt nach einem der Ansprüche 8-11, wobei die Verkapselung Maillard-Reaktionsprodukte umfasst.

13. Produkt, ausgewählt aus der aus einem Lebensmittelprodukt, einem Kosmetikprodukt, einem pharmazeutischen Produkt, einem nutrazeutischen Produkt und einem Industrieprodukt bestehenden Gruppe, wobei das Produkt das Produkt nach einem der Ansprüche 8-12 umfasst.

## Revendications

1. Procédé de préparation d'un produit encapsulé, comprenant
la réaction d'une solution comprenant au moins une protéine choisie dans le groupe constitué de matières solides de lactosérum, isolat de lactosérum et protéine de soja, et au moins un sucre réducteur choisi dans le groupe constitué du glucose et du maltose à un pH initiale d'au moins environ 10 pour obtenir un degré d'hydrolyse de protéine compris entre environ 1 % et environ 15 %, la réaction étant à une température d'au moins 90 °C ;
combinaison de la solution ayant réagi avec une composition comprenant un matériau de noyau ;
dans lequel la solution ayant réagi forme un agent d'encapsulation sur la composition comprenant le matériau de noyau, et dans lequel
le matériau de noyau comprend un acide gras polyinsaturé choisi dans le groupe constitué de l'acide docosahexaénoïque (DHA), l'acide docosapentaénoïque (DPA), l'acide arachidonique (ARA), l'acide eicosapentaénoïque (EPA), l'acide stéaridonique (SDA), l'acide linolénique (LA), l'acide alpha-linolénique (ALA), l'acide gamma-linolénique (GLA), l'acide linolénique conjugué (CLA) et des mélanges de ceux-ci.

2. Procédé de la revendication 1, dans lequel le degré d'hydrolyse de protéine est compris entre environ 2 % et environ 10 %.

3. Procédé de l'une quelconque des revendications 1 à 2, dans lequel la réaction est conduite pendant environ une heure.

4. Procédé de l'une quelconque des revendications 1 à 3, dans lequel l'hydrolyse de protéine est non enzymatique.

5. Procédé de l'une quelconque des revendications 1 à 4, dans lequel le pH initial est choisi dans le groupe constitué d'un pH compris entre 10 et 14, un pH compris entre 11 et 12, et un pH compris entre 10 et 11.

6. Procédé de l'une quelconque des revendications 1 à 5, comprenant en outre l'application d'un deuxième agent d'encapsulation sur le produit encapsulé.

7. Procédé de la revendication 6, dans lequel l'application d'un deuxième agent d'encapsulation est un agent de grelonage.

8. Produit encapsulé, comprenant :
une composition comprenant un matériau de noyau de composé labile choisi dans le groupe constitué d'un acide gras polyinsaturé choisi dans le groupe constitué de l'acide docosahexaénoïque (DHA), l'acide docosapentaénoïque (DPA), l'acide arachidonique (ARA), l'acide eicosapentaénoïque (EPA), l'acide stéaridonique (SDA), l'acide linolénique (LA), l'acide alpha-linolénique (ALA), l'acide gamma-linolénique (GLA), l'acide linolénique conjugué (CLA) et des mélanges de ceux-ci ; et
un agent d'encapsulation sur la composition, l'agent d'encapsulation étant formé de protéine hydrolisée ayant un degré hydrolyse de protéine compris entre environ 1 % et environ 15 % et de produits de caramélisation,
l'agent d'encapsulation pouvant être obtenu par réaction d'une solution comprenant une protéine choisie dans le groupe constitué de matières solides de lactosérum, isolat de lactosérum et protéine de soja, et au moins un sucre réducteur choisi dans le groupe constitué du glucose et du maltose à un pH initiale d'au moins environ 10 pour obtenir un degré d'hydrolyse de protéine compris entre environ 1 % et environ 15 %, la réaction étant à une température d'au moins environ 90 °C.

9. Produit encapsulé de la revendication 8, la réaction étant conduite pendant au moins environ une heure.

10. Produit encapsulé de l'une quelconque des revendications 8 à 9, le pH initial étant choisi dans le groupe constitué d'un pH compris entre 10 et 14, un pH compris entre 11 et 12, et un pH compris entre 10 et 11.

11. Produit encapsulé de l'une quelconque des revendications 8 à 10, le degré d'hydrolyse de de protéine étant compris entre environ 2 % et environ 10 %.

12. Produit encapsulé de l'une quelconque des revendications 8 à 11, l'agent d'encapsulation comprenant des produits de réaction de Maillard.

13. Produit choisi dans le groupe constitué d'un produit alimentaire, un produit cosmétique, un produit pharmaceutique, un produit nutraceutique, et un produit industriel, le produit comprenant le produit de l'une quelconque des revendications 8 à 12.
